# EUROPEAN PATENT SPECIFICATION

(11) **EP 2 613 839 B1**
(45) Date of publication and mention of the grant of the patent: **12.12.2018**
(21) Application number: 11776928.1
(22) Date of filing: 09.09.2011
(51) Int. Cl.: A61M 16/12, A61K 9/72, A61K 33/02, A61M 16/10, A61M 16/08, A61M 15/02

(54) **COMPOSITIONS, METHODS AND ARTICLES CONCERNING PHARMACEUTICAL NITRIC OXIDE AND CONTROLLED PATIENT DELIVERY SYSTEMS THEREOF**
ZUSAMMENSETZUNGEN, VERFAHREN UND ARTIKEL IM ZUSAMMENHANG MIT PHARMAZEUTISCHEM STICKOXID UND GESTEUERTE FREISETZUNGSSYSTEME FÜR PATIENTEN
COMPOSITIONS, PROCÉDÉS ET ARTICLES CONCERNANT L'OXYDE NITRIQUE PHARMACEUTIQUE ET SYSTÈMES D'ADMINISTRATION CONTRÔLÉE D'OXYDE NITRIQUE PHARMACEUTIQUE À UN PATIENT

(30) Priority: 10.09.2010 US 381875 P
(43) Date of publication of application: 17.07.2013
(73) Proprietor: INO Therapeutics LLC, Hampton, NJ 08827 (US)
(72) Inventor: DUMPIT, Ronald F., Bainbride Island, Washington 98110 (US); DECKWERTH, Thomas L., Seattle, Washington 98115 (US); WINTNER, Edward, Belmont, Massachusetts 02478 (US); MONTGOMERY, Frederick J., Sun Prairie, Wisconsin 53590 (US); BATHE, Duncan P., Fitchburg, Wisconsin 53711 (US)
(74) Representative: Jones, Nicholas Andrew
(86) International application number: PCT/US2011/051106
(87) International publication number: WO 2012/034089

(56) References cited:
- WO-A1-92/10228
- WO-A2-03/020211
- US-A- 5 827 420
- US-A1- 2007 190 184

## Description

### TECHNICAL FIELD

Aspects of the present invention relate to an apparatus comprising a tape constructed from a porous hydrophilic polymer substrate having pores, an average pore size, and, a substrate thickness, and, a particulate reductant disposed within the pores and on a surface of the tape forming a film, the particulate reductant having an average particle size, and, the film having a surface density of particulate reductant and a film thickness.

### BACKGROUND OF THE INVENTION

A portable, bedside reactor for generating pharmaceutical nitric oxide from reacting nitrite solution and a reductant is described in commonly-assigned U.S. Patent Application Publication No. 2007/0190184 A1 entitled "Method and Apparatus for Generting Nitric Oxide for Medical Use" that was filed on February 16, 2006 as Serial No. 1 1/355,670 to Montgomery FJ et al. It would be desirable to provide alternative means to generate nitric oxide for deliver to a patient in addition to the reactor described in U.S. Patent Application Publication No. 2007/0190184 A1.

### SUMMARY OF THE INVENTION

The present invention is defined in the appended claims. The apparatus comprises a tape constructed from a porous hydrophilic polymer substrate having pores, an average pore size, and, a substrate thickness, and, a particulate reductant disposed within the pores and on a surface of the tape forming a film, the particulate reductant having an average particle size, and, the film having a surface density of particulate reductant and a film thickness.

The reductant comprises one or more compounds according to the following structures: 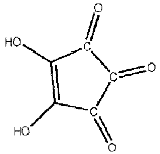 (croconic acid), 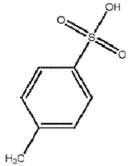 (p-toluene-sulfonic acid), and 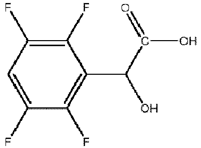 (2,3,5,6-tetrafluoro-mandelic acid), or, a salt, ester, anhydride, crystalline form, amorphous form thereof, or a combination thereof, or, a combination of reductants thereof.

In an exemplary embodiment, the reductant comprises croconic acid according to the structure, 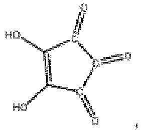 or a salt thereof.

In another exemplary embodiment, the porous hydrophilic polymer is a polyamide nylon.

In another exemplary embodiment, the average pore size is in the range of 0.1-20 µm, and, the substrate thickness is in the range of 50-200 µm.

In another exemplary embodiment, the average pore size is in the range of 0.22-5.0 µm, and, the substrate thickness is in the range of 65-125 µm.

In another exemplary embodiment, the average particle size is in the range of 1-32 µm, and, the density of the particulate reductant is in the range of 1-15 M.

In another exemplary embodiment, the average particle size is in the range of 1-20 µm, and, the density of the particulate reductant is in the range of 10-12 M.

In another exemplary embodiment, the particulate reductant is bound to the substrate by an in situ reaction between the particulate reductant, an alcohol and the substrate.

In another exemplary embodiment, the average particle size is in the range of 20-32 µm.

In another exemplary embodiment, the surface density is in the range of 2-25 µmoles/cm², and, the film thickness is in the range of 8-40 µm.

In another exemplary embodiment, the surface density is in the range of 8-9 µmoles/cm², and, the film thickness is in the range of 10-23 µm.

In another exemplary embodiment, the substrate is a spoolable tape.

The tape may be made by applying a suspension comprising the particulate reductant and an alcohol selected from methanol, ethanol, isopropyl or a combination thereof to the substrate, wherein the particulate reductant is crystalline, and, wherein the density of the particulate reductant is in the range of 1-15 M or 10-12 M. The alcohol may comprise USP absolute methanol, USP absolute ethanol or a mixture thereof. The suspension may comprise in the range of 1-50 mg/mL or 1-20 mg/mL of the particulate reductant. The suspension may further comprise 0.01-0.1 % v/v glycerol and 1-3% v/v starch particulate reductant may comprise croconic acid according to the structure 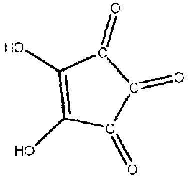 or, a salt thereof.

An exemplary method of making the tape may include providing the tape constructed from the porous hydrophilic polymer substrate having pores, the average pore size, and, the substrate thickness, and, applying to the substrate the suspension comprising the particulate reductant having the average particle size and the alcohol selected from methanol, ethanol, isopropyl or a combination thereof.

In an example, the alcohol comprises USP absolute methanol, USP absolute ethanol or a mixture thereof.

In another example, the suspension comprises in the range of 1-50 mg/mL or 1-20 mg/mL of the particulate reductant.

In another example, the reductant comprises croconic acid according to the structure 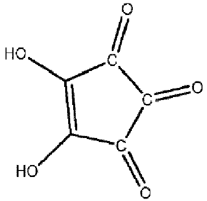 or a salt thereof.

In another example, the porous hydrophilic polymer is a polyamide nylon, and, the substrate is a spoolable tape.

The apparatus also comprises a liquid pulsing device adapted to pulse liquid droplets of a pharmaceutical liquid source comprising water, nitrite ions (NO₂⁻), a viscosity agent, and, a non-detergent surfactant onto the tape generating pulses of nitric oxide gas.

In an exemplary embodiment of the liquid source, the non-detergent surfactant is nonionic, and, the water is deionized and millipore-filtered.

In another exemplary embodiment of the liquid source, the non-detergent, nonionic surfactant comprises a nonylphenol ethoxylate.

In another exemplary embodiment of the liquid source, it comprises 0.01-5.0% v/v of the nonylphenol ethoxylate.

In another exemplary embodiment of the liquid source, it comprises 0.01-1.0% v/v of the nonylphenol ethoxylate.

In another exemplary embodiment of the liquid source, the nonylphenol ethoxylate is a compound according to the structure 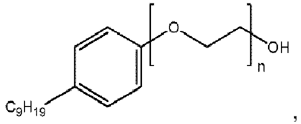 wherein the average molecular weight is around 616.

In another exemplary embodiment of the liquid source, the viscosity agent comprises polyethylene glycol having a molecular weight in the range of 3500-20,000.

In another exemplary embodiment of the liquid source, the viscosity agent comprises polyethylene glycol having a molecular weight within the range of around 4400-4600.

In another exemplary embodiment of the liquid source, it comprises 1-20% v/v of the polyethylene glycol.

In another exemplary embodiment of the liquid source, it comprises 4-6% v/v of the polyethylene glycol.

In another exemplary embodiment of the liquid source, it has a viscosity in the range of 8-15 cp.

The liquid source may be made by dissolving NaNO₂ to produce 0.1-10 M NO₂⁻.

In another example the liquid source may be made by dissolving NaNO₂ to produce 1-6 M NO₂⁻.

In another exemplary embodiment of the liquid source, it is substantially free of dissolved O₂.

The invention is a medical device or apparatus for making pharmaceutical nitric oxide gas comprising an enclosure defining a reactor chamber having a gas inlet port and a gas outlet port, the inlet port adapted to receive an air flow (the air flow may further be mixed with a stream of O₂ and/or N₂ gas), and, the gas outlet port adapted to discharge a gas flow; a tape constructed from the porous hydrophilic polymer substrate having pores, the average pore size, and, the substrate thickness; and, the particulate reductant disposed within the pores and on the surface of the tape forming the film, the particulate reductant having the average particle size, and, the film having the surface density of the particulate reductant and the film thickness, the tape disposed within the reaction chamber, and, the tape wound around first and second cylinders of a spooling mechanism, the spooling mechanism adapted to spool the tape within the reaction chamber; and, a liquid pulsing device adapted to pulse liquid droplets of the liquid source comprising the water, NO₂⁻, viscosity agent, and, non-detergent surfactant onto the tape generating pulses of the nitric oxide gas. The reductant comprises one or more of croconic acid, p-toluene sulfonic acid and 2,3,5,6-tetrafluoromandelic acid.

In an exemplary embodiment of the medical device or apparatus, the liquid pulsing device is in liquid communication with a liquid inlet port disposed on the enclosure.

In an exemplary embodiment of the medical device or apparatus, the spooling mechanism is a cassette.

In another exemplary embodiment of the medical device or apparatus, the liquid pulsing device is a piezoelectric ink jet printhead.

In another exemplary embodiment of the medical device or apparatus, the piezoelectric ink jet printhead is adapted to generate liquid droplets having a volume in the range of 10-100 picoliters.

In another exemplary embodiment of the medical device or apparatus, the piezoelectric ink jet printhead is adapted to generate pulses of droplets at a frequency up to 12 MHz.

In another exemplary embodiment of the medical device or apparatus, the piezoelectric ink jet printhead is adapted to generate 10-100 nL per pulse of the liquid source.

In another exemplary embodiment of the medical device or apparatus, the piezoelectric ink jet printhead is adapted to generate 25-75 nL per pulse of the liquid source.

In another exemplary embodiment of the medical device or apparatus, the piezoelectric ink jet printhead is adapted to generate around 48 nL per pulse of the liquid source.

An exemplary method of generating pulses of pharmaceutical nitric oxide may comprise atomizing the liquid source comprising water, NO₂⁻, viscosity agent, and, non-detergent surfactant generating liquid droplets; pulsing the liquid droplets onto a substrate constructed from the porous hydrophilic polymer having pores, the average pore size, and, the substrate thickness, and, the particulate reductant disposed within the pores and on the surface of the substrate forming the film, the particulate reductant having the average particle size, and, the film having the surface density of particulate reductant and the film thickness; and, generating the pulses of pharmaceutical nitric oxide.

In an example of the method of generating pulses of pharmaceutical nitric oxide, the liquid droplets having a volume in the range of 10-100 picoliters. The pulses of liquid droplets may be generated at a frequency up to 12 MHz, and, 1 0-100, 25-75 or 48 nL per pulse of the liquid source may be generated.

In another example of the method of generating pulses of pharmaceutical nitric oxide, the pulses of generated pharmaceutical nitric oxide are mixed with an air flow to produce a gas flow comprising nitric oxide at a concentration in the range of 0.1-900 ppm.

An exemplary method of controlled administration of pharmaceutical nitric oxide to the lung of a human patient in need thereof by inhalation may comprise continuously, spontaneously or intermittently atomizing the liquid source comprising water, NO₂⁻, viscosity agent, and, non-detergent surfactant generating the liquid droplets, the molarity of NO₂⁻ being predetermined; continuously, spontaneously or intermittently pulsing the liquid droplets onto the substrate constructed from the porous hydrophilic polymer having pores, the average pore size, and, the substrate thickness, and, the particulate reductant disposed within the pores and on the surface of the substrate forming the film, the particulate reductant having the average particle size, and, the film having the surface density of particulate reductant and the film thickness; generating the pulses of pharmaceutical nitric oxide gas; controlling the amount of nitric oxide generated in the pulses by controlling the frequency of droplets pulsed onto the film; controlling the air flow adapted to mix with the pulses of pharmaceutical nitric oxide gas producing a predetermined concentration of nitric oxide in a gas flow; and, administering the gas flow to the lung of the human patient in need thereof by inhalation.

In an example of the method of controlled administration, the method further comprises filtering the air flow to remove moisture and airborne contaminants and/or treating the air flow passing through the nitric oxide generating device to remove O₂.

In another example of the method, it further includes sensing the concentration of nitric oxide in the gas flow, sensing the patient's breath, and, triggering delivery of the gas flow to the patient by inhalation.

The method may further include ventilating the patient creating an inspiratory flow and an expiratory flow.

The method may still further include ventilating the patient creating an inspiratory flow and an expiratory flow, the gas flow mixing with the inspiratory flow; sensing the concentration of the nitric oxide in the gas flow; and, sensing the concentration of nitric oxide in the inspiratory flow before said mixing with the gas flow.

The method may still further include ventilating the patient creating the inspiratory flow and expiratory flow, the gas flow mixing with the inspiratory flow; sensing the concentration of nitric oxide in the inspiratory flow before said mixing with the gas flow; and, sensing the concentration of nitric oxide in the inspiratory flow after said mixing with the gas flow.

The method may also include setting a predetermined concentration of nitric oxide to be administered to the lung of the human patient.

In another example of the method, the gas flow administered to the patient's lungs comprises nitric oxide at a concentration in the range of 0.1-900 ppm.

Another exemplary method of controlled administration of pharmaceutical nitric oxide to the lung of a human patient in need thereof by inhalation may comprise continuously, spontaneously or intermittently atomizing the liquid source comprising water, NO₂⁻, viscosity agent, and, non-detergent surfactant generating the liquid droplets, the molarity of NO₂⁻ being predetermined; continuously, spontaneously or intermittently pulsing the liquid droplets onto the substrate constructed from the porous hydrophilic polymer having pores, the average pore size, and, the substrate thickness, and, the particulate reductant disposed within the pores and on the surface of the substrate forming the film, the particulate reductant having the average particle size, and, the film having the surface density of particulate reductant and the film thickness; generating the pulses of pharmaceutical nitric oxide; controlling the amount of nitric oxide generated in the pulses by controlling the frequency of droplets pulsed onto the film; ventilating the patient creating the inspiratory flow and the expiratory flow; controlling the inspiratory flow, the inspiratory flow mixing with the pulses of pharmaceutical nitric oxide gas producing a predetermined concentration of nitric oxide in the inspiratory flow; sensing of the concentration of nitric oxide in the inspiratory flow before mixing the gas flow with the inspiratory flow; sensing of the concentration of nitric oxide in the inspiratory flow after mixing the gas flow with the inspiratory flow; setting a predetermined concentration of nitric oxide in the inspiratory flow occurring after mixing the gas flow with the inspiratory flow; and, administering the inspiratory flow containing the predetermined concentration of nitric oxide to the lung of the human patient. In an example, the gas flow administered to the patient's lungs comprises nitric oxide at a concentration in the range of 0.1-900 ppm.

The medical device or apparatus may be used with a delivery system for controlled administration of pharmaceutical nitric oxide gas to the lung of a human patient in need thereof by inhalation, wherein the device or apparatus and the delivery system comprises the enclosure defining the reactor chamber having the gas inlet port and the gas outlet port, the inlet port adapted to receive an air flow, and, the gas outlet port adapted to discharge a gas flow; the tape constructed from the porous hydrophilic polymer substrate having pores, the average pore size, and, the substrate thickness, and, the particulate reductant disposed within the pores and on the surface of the tape forming the film, the particulate reductant having the average particle size, and, the film having the surface density of particulate reductant and the film thickness, the tape disposed within the reaction chamber, and, the tape wound around first and second cylinders of the spooling mechanism, the spooling mechanism adapted to spool the tape within the reaction chamber; the liquid pulsing device adapted to pulse liquid droplets of the liquid source comprising water, NO₂⁻, viscosity agent, and, non-detergent surfactant onto the tape generating the pulses of nitric oxide gas; an air pump adapted to pump the air flow to the inlet port, the air flow mixing with the pulses of nitric oxide gas producing the gas flow adapted to exit the reactor chamber through the gas outlet port; and, a controller system in electronic communication with and adapted to control the air pump, liquid pulsing device, and, spooling mechanism, respectively. The reductant comprises one or more of croconic acid, p-toluene sulfonic acid and 2,3,4,6-tetrafluoromandelic acid.

In an example of the medical device and delivery system, it further includes an air filter adapted to remove moisture and airborne contaminants from the air flow and/or a membrane separator adapted to remove 02 from the air flow passing through the nitric oxide generating device.

In another example of the medical device and delivery system, the spooling mechanism is a cassette, and, a nasal cannula, gas mask or endotracheal tube adapted to administer the gas flow is included.

In another example of the medical device and delivery system, it further includes a nitric oxide sensor adapted to sense the concentration of nitric oxide in the gas flow, the controller system being in electronic communication with said nitric oxide sensor.

In another example of the medical device and delivery system, it further includes a breath trigger sensor adapted to sense the patient's breath, the controller system being in electronic communication with the breath trigger sensor.

In another example of the medical device and delivery system, it further includes a ventilator circuit in communication with the gas flow, the ventilator circuit including a mechanical ventilator, an inspiratory limb having an inspiratory flow, and, an expiratory limb having an expiratory flow.

In another example of the medical device and delivery system, it further includes the ventilator circuit including the mechanical ventilator, the inspiratory limb, and, the expiratory limb, the gas flow mixing with the inspiratory flow; and, a nitric oxide sensor in electronic communication with the controller system and adapted to sense the concentration of nitric oxide in the inspiratory flow before such mixing with the gas flow.

In another example of the medical device and delivery system, it further includes the ventilator circuit in communication with the gas flow, the ventilator circuit including the mechanical ventilator, the inspiratory limb having the inspiratory flow, and, the expiratory limb having the expiratory flow, the gas flow mixing with the inspiratory flow; a sensor in electronic communication with the controller system and adapted to sense the concentration of nitric oxide in the inspiratory flow before such mixing with the gas flow; and, another sensor in electronic communication with the controller system and adapted to sense the concentration of nitric oxide in the inspiratory flow after such mixing with the gas flow.

In another example of the medical device and delivery system, it further includes a nitric oxide concentration set in electronic communication with the controller system and adapted to set a predetermined concentration of nitric oxide to be administered to the patient's lung, such as a nitric oxide concentration in the range of 0.1 ppm to 900 ppm.

The medical device or apparatus may be used with another delivery system for controlled administration of pharmaceutical nitric oxide gas to the lung of a human patient in need thereof by inhalation, wherein the device or apparatus and the delivery system includes the ventilator circuit including the mechanical ventilator, the inspiratory limb having the inspiratory flow, and, the expiratory limb having the expiratory flow; the enclosure defining the reactor chamber disposed in the inspiratory limb, the enclosure having the gas inlet port, the gas outlet port, and, an inspiratory limb inlet port, the inlet port adapted to receive the air flow, the inspiratory limb inlet port adapted to receive the inspiratory flow, and, the gas outlet port adapted to discharge the gas flow in the inspiratory limb; the tape constructed from: the porous hydrophilic polymer substrate having pores, the average pore size, and, the substrate thickness, and, the particulate reductant disposed within the pores and on the surface of the tape forming the film, the particulate reductant having the average particle size, and, the film having the surface density of particulate reductant and the film thickness, the tape disposed within the reaction chamber, and, the tape wound around the first and second cylinders of the spooling mechanism, the spooling mechanism adapted to spool the tape within the reaction chamber; the liquid pulsing device adapted to pulse the liquid droplets of the liquid source containing the water, NO₂⁻, viscosity agent, and, non-detergent surfactant onto the tape generating the pulses of nitric oxide gas; the air pump adapted to pump the air flow to the inlet port, the air flow mixing with the pulses of nitric oxide gas and inspiratory flow producing the gas flow adapted to exit the reactor chamber through the gas outlet port; a nitric oxide sensor adapted to sense the concentration of nitric oxide in the inspiratory limb before such mixing of the air flow, pulses of nitric oxide and inspiratory flow; another nitric oxide sensor adapted to sense the concentration of nitric oxide in the inspiratory limb after mixing of the air flow, pulses of nitric oxide and inspiratory flow; and, the controller system in electronic communication with and adapted to control the air pump, liquid pulsing device, and, spooling mechanism, respectively, and, in electronic communication with the first and second nitric oxide sensors, respectively. The reductant comprises one or more of croconic acid, p-toluene sulfonic acid and 2,3,4,6-tetrafluoromandelic acid.

In an example of this medical device and delivery system, it further includes the air filter adapted to remove moisture and airborne contaminants from the air flow and/or the membrane separator adapted to remove O₂ from the air flow. The spooling mechanism may also be cassette, and, a nasal cannula, gas mask or endotracheal tube adapted to administer the gas flow may be included.

In another example of this medical device and delivery system, it further includes the nitric oxide concentration set in electronic communication with the controller system, the controller system adapted to control a predetermined set concentration of nitric oxide administered to the patient's lung.

In another example of this medical device and delivery system, nitric oxide concentration in the range of 0.1 ppm to 900 ppm is administered to the patient's lung.

### BRIEF DESCRIPTION OF THE DRAWINGS

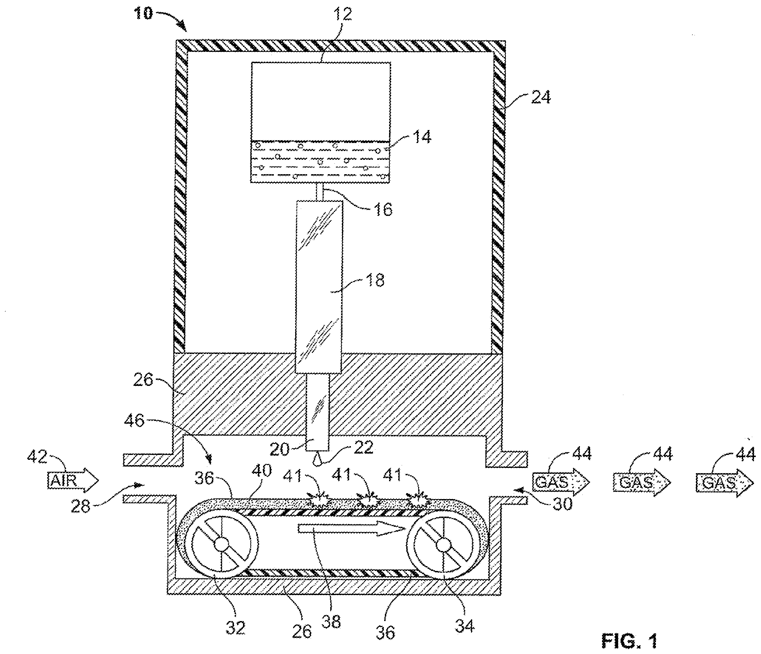

FIG. 1 is a schematic illustration of a nitric oxide generating reactor, whereby a nitrite solution containing a non-detergent surfactant is atomized by a micro pump and nozzle producing nitrite-containing droplets, whereby the droplets contact a hydrophilic polymeric tape that has been treated (i.e., coated and/or impregnated) with a particulate reductant compound, whereby the nitrite (NO₂⁻) reacts with the particulate reductant producing gas pulses of nitric oxide that mix with an air flow from an inlet to the reactor chamber producing pulses of a gas flow being a mixture of air and nitric oxide that exits through an outlet from the reactor, and, whereby the hydrophilic tape is wound around two spools that are part of a cassette assembly.
FIG. 2 is a disassembled view of another nitric oxide generating reactor assembly having a micro dispensing valve (e.g., an ink jet assembly), a top plate, an insert, a cassette and a bottom plate.
FIG. 3 is a schematic illustration of a direct, open-loop, controlled nitric oxide delivery system.
FIG. 4 is a schematic illustration of a direct, open-loop and controlled nitric oxide delivery system connected to a ventilated patient.
FIG. 5 is a schematic illustration of another direct, open-loop and controlled nitric oxide delivery system connected to a ventilated patient.
FIG. 6 is a schematic illustration of yet another direct, open-loop and controlled nitric oxide delivery system connected to a ventilated patient.
FIG. 7 is a schematic illustration of a direct, closed-loop and controlled nitric oxide delivery system connected to a ventilator.
FIG. 8 is a bar graph showing nitric oxide release from various particulate (i.e., powdered solids) reductant compounds exposed to droplets of 6M NaNO₂. The amount of NO measured as area under the curve (AUC), the maximum NO concentration achieved, and the rate at which NO is being released are compared for the reductant compounds.
FIG. 9 is a bar graph showing comparative nitric oxide release composite scores by reacting 6M NaNO₂ droplets with various particulate reductant compounds. Release scores are calculated as AUC/([time to 90% AUC]^ 0.7).

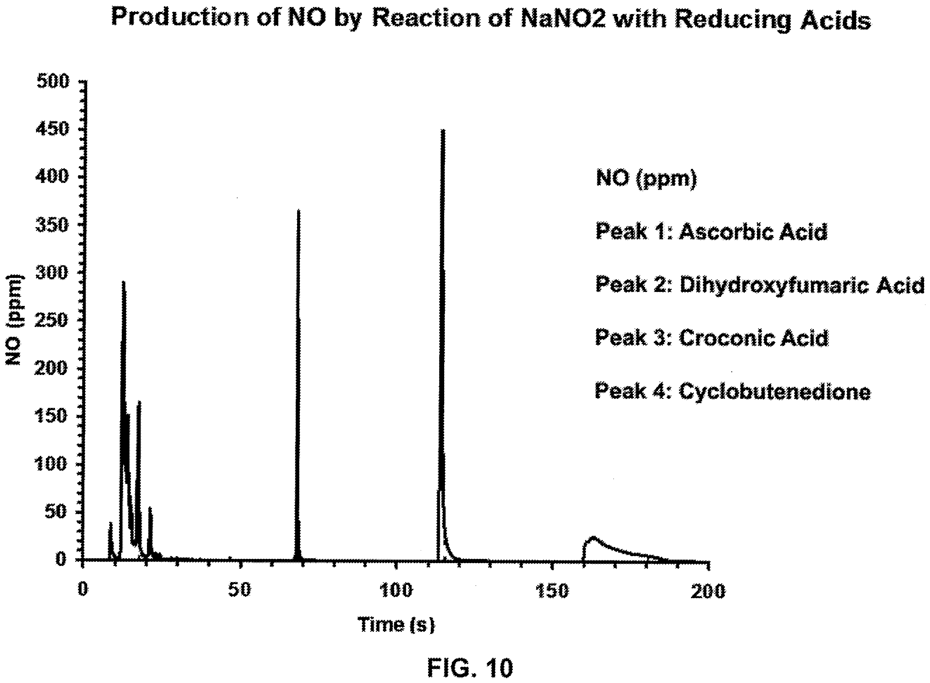

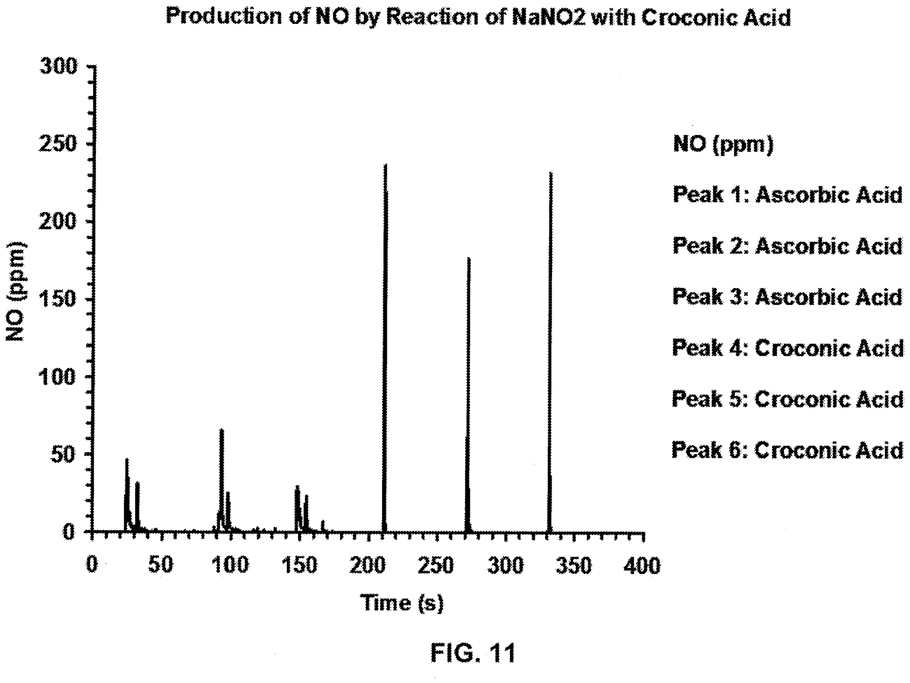

FIG. 10 is a graph showing time courses of nitric oxide concentrations produced by reacting NaNO2 droplets with selected reducing acids. NO release peaks are labeled from left to right as numbers 1-4. The same numbering convention applies to FIGS. 11-16.
FIG. 11 is a graph showing the time courses of nitric oxide concentrations produced by reacting NaNO₂ droplets with particulate ascorbic acid and croconic acid. NO concentrations are not corrected for dilution of the gas stream prior to NO measurement.
FIGS. 12 and 13 are graphs showing the time course of nitric oxide concentrations produced by reacting a NaNO₂ solution with powdered ascorbic acid or croconic acid to demonstrate the differences in the release characteristics. FIG. 13 shows a detail of FIG. 12.
FIG. 14 is a graph showing the time course of nitric oxide release produced by reaction of NaNO₂ droplets with finely ground particulate croconic acid which was formulated as suspension in methanol and additives of 0.05% v/v glycerol and 2% v/v starch and coated onto MAGNA® nylon tape. NO concentrations are not corrected for dilution of the gas stream prior to NO measurement.
FIG. 15 is a graph showing the time courses of nitric oxide release produced by reaction of NaNO₂ droplets with particulate croconic acid of particle size < 20 µm which was formulated as suspension in ethanol by 10 min of sonication with additives of 0.05% v/v glycerol and 2% v/v starch present and coated onto MAGNA® nylon tape.
FIG. 16 is a graph of the time course of nitric oxide release produced by reaction of NaNO₂ droplets with particulate croconic acid which was formulated as suspension in isopropanol and coated onto MAGNA® nylon tape.
FIG. 17 shows the chemical reaction of ascorbic acid with NaNO₂. Two equivalents of ascorbic acid act as acid and one equivalent as reductant, consuming three moles of ascorbic acid per mole of nitrite.
FIGS. 18A and 18B show the chemical reaction between croconic acid and NaNO₂. Two moles of croconic acid are consumed per mole of nitrite.
FIGS. 19 and 20 are bar graphs comparing the release of NO by bulk reactions of ascorbic acid or croconic acid with NaNO₂ solution. Compared to ascorbic acid, croconic acid is more efficient at producing NO gas on a molar basis, consistent with the more favorable stoichiometry of the reaction.
FIG. 21 is a graph demonstrating nitric oxide generation by the device of FIG 2. NO is generated in direct proportion to the amount of nitrite-containing fluid deposited on a MAGNA® nylon tape coated and impregnated with particulate croconic acid.

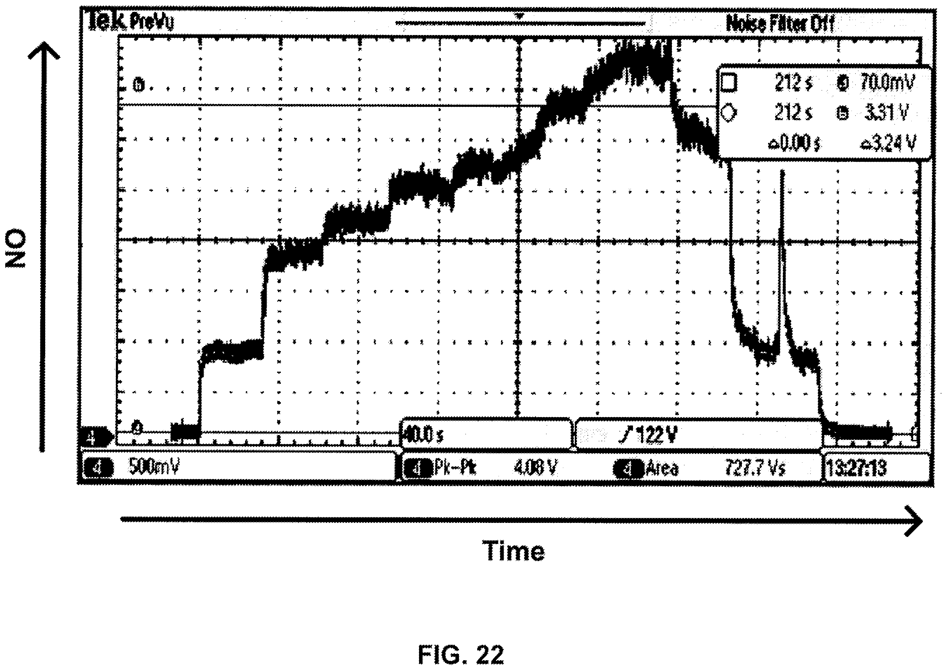

FIG. 22 shows an example of the raw NO release data from a croconic acid-coated nylon tape by the device of FIG 2. In this experiment, sodium nitrite deposition increased in a step-wise fashion over time, leading to increases in NO production by the device.

### DETAILED DESCRIPTION

As used herein, the phrase "gas flow" refers to a flow of gases (i.e., gas flow 44 as shown in FIG. 1) exiting the reaction chamber 46 through the outlet 30, whereby the flow of gas or gases may be pulsed, constant (i.e. steady state) or variable, and whereby the flow of gas or gases may be intermittent, spontaneous or continuous. For example, the flow of gas or gases may be (1) constant in flowrate and NO concentration, (2) constant in flowrate and pulsed in NO concentration (trigger used to pulse NO), or (3) pulsed in flowrate and pulsed in NO concentration (trigger used to pulse). While the generation of NO is pulsed, a constant (i.e., non-pulsed) flowrate and/or concentration of NO may be achieved by incorporating system and process controllers known in the art to control the droplet frequency in a constant or pulsed manner.

As used herein, the phrase "air flow" refers to a flow of air that may be constant or varied to achieve a predetermined concentration of NO in the gas flow.

All %'s recited herein are in terms of % by volume (i.e., % v/v) unless otherwise indicated.

Shown in FIG. 1 is a schematic view of a nitric oxide pulse generator 10 having on the outside thereof a plastic housing 24 is affixed to a metallic reactor 26. A reservoir 12 contains an aqueous nitrite solution 14 made by dissolving sodium nitrite (NaNO₂) in water producing nitrite ions (NO₂⁻). The water may be deionized. The water may also be purified by a millipore purification system. The water may also be deoxygenated, whereby the water has an O₂ content < 0.5 µM.

The aqueous nitrite solution 14 also contains a viscosity agent and a non- detergent surfactant. The non-detergent surfactant may be a TERGITOL™ nonylphenol ethoxylate surfactant according to the structure 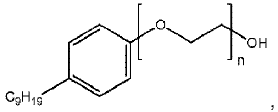 such as NP-9 having a molecular weight of 616, a HLB of 12.9 (calculated), a surface tension of 30 Dynes/cm (0.1 % v/v aqueous solution at 25°C), and, a viscosity of 243 cp at 25°C. The viscosity agent may be a polyethylene glycol having a molecular weight in the range of 2500-20,000 or 4400-4600. The viscosity of the aqueous nitrite solution may be in the range of 8 cp to 15 cp.

Further as shown in FIG. 1, a micro pump 18 draws the solution 14 from the reservoir 12 through a tube 16 and through a micro nozzle 20 producing pulses of droplets 22. The end of the nozzle 20 is disposed within a reaction chamber 46 defined by the reactor 26. The reactor 26 also includes an inlet 28 for receiving a flow of air 42. The reactor 26 further includes an outlet 30, whereby pulses of the gas flow 44 comprising a mixture of air and nitric oxide gas exit the reactor 26 through the outlet 30.

Pulses of nitric oxide within the reaction chamber 46 are produced by pulses of chemical reaction bursts 41 on the surface of a porous and hydrophilic nylon tape 36 coated and impregnated with a particulate reductant 40. The particulate reductant may be micronized. The coated/impregnated tape 36/40 moves in direction of the arrow 38 perpendicular and transverse to the nozzle 20 and droplets 22 by actuation of opposing scrolls 32, 34. The generator 10 may be controlled by an electronic process control device, such as a computer.

The tape 36 (also referred to herein as a substrate) may be constructed from a porous and hydrophilic polyamide nylon. Exemplary tapes and substrates are constructed from hydrophilic polyamide nylons commercially available from Osmonics under the brand name MAGNA™ Nylon Membrane, which is a supported, naturally hydrophilic membrane designed to wet out evenly and retain its superior strength during use in general filtration and medical assays. MAGNA™ nylons are available in various thickness in the range of 65-1 25 µm and pore sizes including 0.1, 0.22, 0.45, 0.6, 0.8, 1.2, 5.0, 10.0 and 20.0 µm.

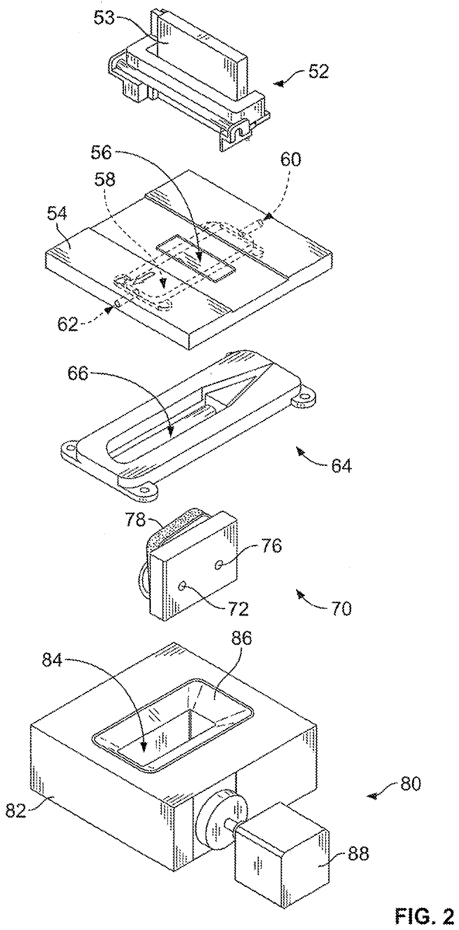

Shown in FIG. 2 is a disassembled view of another nitric oxide pulse generator, which may also be controlled by an electronic process control device, such as a computer. One component is an inkjet assembly 52 having a piezoelectric ink jet cartridge 53. The inkjet assembly 52 may be a DIMATIX™ inkjet available from FUJIFILM. The inkjet assembly 52 mounts on a top plate 54 such that the inkjets emit pulses of NO₂⁻ containing droplets through droplet aperture 56. A recess 58 in the top plate 54 defines a reaction chamber having an air flow inlet 60 and an outlet 62 for the pulses of a gas mixture of air and nitric oxide.

Another component is a reaction chamber insert 64 mounted to the top plate 54. The insert 64 has a recessed aperture 66 that, in combination with the recess 58, defines, in part, a reaction chamber.

Further as shown in FIG. 2, another component is a cassette 70 having opposing spools 72,76 for spooling a porous and hydrophilic nylon tape coated and impregnated with the particulate reductant 78. The particulate reductant may be micronized, crystalline or amorphous croconic acid. The cassette 70 is disposed in a cassette chamber 84 in a reactor 80. The insert 64 is mounted to the bottom plate 82, whereby all of the components are sealed to provide a sealed reaction chamber 58. A recess 86 is provided that, in combination with the recess 58 and aperture 66, define the reaction chamber. A motor 88 turns the cassette spool 72. The motor 88 is controlled by a controller system (not shown) to advance the tape 78 providing un-reacted reductant 40 for reacting with the droplets 22, and, to ensure that a predetermined therapeutic concentration of nitric oxide is delivered to the patient.

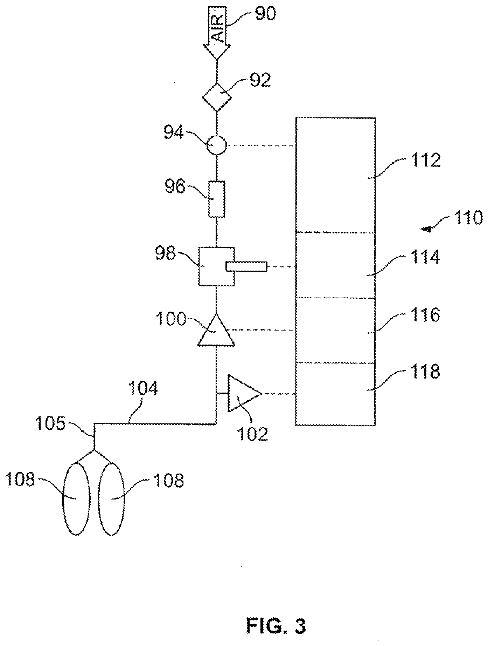

Shown in FIG. 3 is a direct, open-loop, controlled nitric oxide generation and delivery system for continuously, spontaneously or intermittently administering a therapeutic dose or concentration of gaseous nitric oxide to a patient 108 in need thereof. Ambient air 90 is filtered by an air filter 92. The ambient air 90 is pumped by an air pump 94, which can also be located further downstream. The air pump 94 is controlled by an air pump control 112 to assist in controlling the therapeutic dose or concentration of nitric oxide delivered to the patient 108. The filtered air is further filtered by an oxygen (O₂) membrane separator 96 to produce filtered and deoxygenated air, which advantageously eliminates or substantially reduces the risk of NO₂ being generated prior to administration to the patient 108. The oxygen membrane separator 96 is optional such that the air may be oxygenated or deoxygenated.

Further as shown in FIG. 3, the filtered and deoxygenated air flow is fed to a nitric oxide pulse generator 98, which may be similar or identical to the nitric oxide pulse generators shown in FIGS. 1 and 2, whereby variations of such generators may also be employed. A droplet control 114 controls the frequency and/or amplitude of aqueous, deoxygenated, millipore-filtered, nitrite (NO₂⁻) containing solution (that also contains non-detergent surfactant and viscosity agent) delivered to the reductant- impregnated/coated, hydrophilic nylon tape 36/40 (not shown in FIG. 3). Pulses of gas flow containing nitric oxide generated in the generator 98 mix with the filtered and deoxygenated air producing the controlled dose or concentration of nitric oxide in the gas flow, which passes through the conduit 104 to a nasal cannula 105 disposed on the patient 108. The frequency of droplets is a significant determinant of the concentration of nitric oxide in the therapeutic gas administered to the patient 108.

An NO/NO₂ sensor 100 and a corresponding NO/NO₂ sensor monitor 116 are provided to monitor the concentration of NO and NO₂ in the gas flow containing nitric oxide. It is important to continuously monitor the presence of NO₂ where the sensors are disposed because it is desirable to eliminate or minimize to the extent possible the presence of NO₂ throughout the various embodiments. A breath trigger 102 and a breath trigger monitor 118 are provided to sense patient breaths for proper administration of the therapeutic nitric oxide containing gas flow to the patient 108. The breath trigger 102 and breath trigger monitor 118 are optional components. The sensor 100 and monitor 116 also monitor the presence and concentration thereof of any undesired NO₂ in the gas flow.

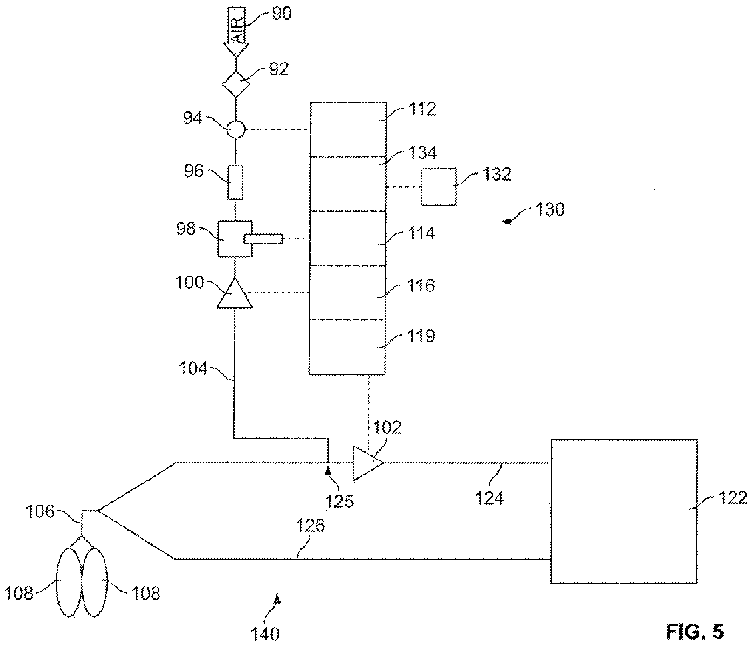

The controller system 110 includes the air pump control 112, droplet control 114, the NO/NO₂ sensor monitor 116, and the breath trigger monitor 118, which control their corresponding components to ensure accurate and controlled administration of the therapeutic nitric oxide containing gas flow to the patient 108. The controller system 110 may be a CPU or any suitable microprocessor and related electronics, whereby the sensors and controls are in electronic communication collectively and individually with their corresponding pneumatic components. The other controller systems 130, 160 shown in FIGS. 5, 6 and 7, including their controls and corresponding sensors, are in electronic communication collectively and individually with corresponding pneumatic components shown therein.

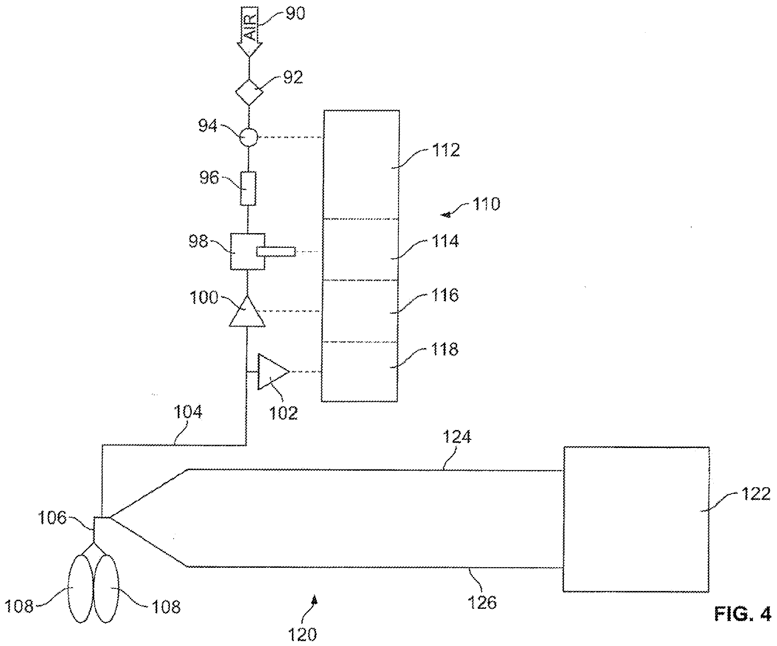

Shown in FIG. 4 is a direct, open-loop, controlled and ventilated nitric oxide generation and delivery system for continuously, spontaneously or intermittently administering a therapeutic dose or concentration of gaseous nitric oxide to the patient 108 in need thereof. Many of the components are the same and arranged identical to that shown in FIG. 3. For example, ambient air 90 is filtered by an air filter 92 and O₂ membrane separator 96 using air pump 94, which can also be located further downstream. The air pump 94 and corresponding air pump control 112 control the therapeutic dose or concentration of nitric oxide delivered to the patient 108.

The filtered and deoxygenated air is fed to a nitric oxide pulse generator 98 (see the generators shown in FIGS. 1 and 2). The droplet control 1 14 controls the frequency of aqueous NO₂⁻ containing solution that is delivered to the reductant-impregnated/coated, hydrophilic nylon tape 36/40 as shown in FIGS. 1 and 2. The frequency of the droplets sets the pulse rate of nitric oxide being generated. Pulses of gaseous nitric oxide mix with the filtered and deoxygenated air producing the controlled dose or concentration of therapeutic nitric oxide containing gas flow, which passes thorough the conduit 104 to a gas mask, endotracheal tube and/or nasal cannula 106 disposed on the patient 108. The NO/NO₂ sensor 100 and a corresponding NO/NO₂ control monitor 116 to monitor the concentration of nitric oxide in the nitric oxide containing gas flow as well as the presence and concentration of undesirable NO₂. The breath trigger sensor 102 and breath trigger monitor 118 ensure proper administration of the therapeutic nitric oxide containing gas flow to the patient 108.

As shown in FIG. 4, the controller system 110 includes the air pump control 112, droplet control 114, NO/NO₂ sensor monitor 116, and breath trigger monitor 118, which control their corresponding components to ensure accurate and controlled administration of the gaseous therapeutic nitric oxide to the patient 108

Further shown in FIG. 4 is a mechanical ventilator circuit 120 including a mechanical ventilator 122, inspiratory limb 124 and expiratory limb 126. The circuit 120 is in gas communication with the conduit 104 to provided ventilated administration of the therapeutic nitric oxide containing gas flow to the patient 108.

Shown in FIG. 5 is a direct, open-loop, controlled and ventilated nitric oxide generation and delivery system for continuously, spontaneously or intermittently administering a therapeutic dose or concentration of gaseous nitric oxide to the patient 108 in need thereof. Many of the components are the same and arranged identical to that shown in FIGS. 3 and 4. For example, ambient air 90 is filtered by air filter 92 by air pump 94, which can also be located further downstream. The air pump 94 and corresponding air pump control 112 control the therapeutic dose or concentration of nitric oxide containing gas flow delivered to the patient 108.

The ambient air 90 is also filtered by an O₂ membrane separator 96. The filtered and deoxygenated air is fed to the nitric oxide pulse generator 98, which is controlled by a droplet control 114. The droplet control 114 controls the frequency of aqueous NO₂⁻ containing solution that is delivered to the reductant-impregnated/coated hydrophilic tape 36/40. A NO/NO₂ sensor 100 is disposed immediately downstream from the generator 98 to measure the concentration of NO in the gas flow as well as the presence and concentration of any undesired NO₂. The NO/NO₂ sensor 100 communicates such data to the NO sensor monitor 116.

The controller system 130 includes the air pump control 112, droplet control 114, the NO/NO₂ sensor monitor 116, and NO₂ control 119, which control their corresponding components to ensure accurate and controlled administration of the therapeutic nitric oxide containing gas flow to the patient 108 as well as detect the presence of NO₂ in the inspiratory limb 124. The controller system 130 also includes a nitric oxide concentration set 132 and a corresponding nitric oxide concentration set control 134 for setting a predetermined concentration or dose of nitric oxide administered to the patient in the therapeutic nitric oxide containing gas flow.

The mechanical ventilator circuit 140 includes the mechanical ventilator 122, inspiratory limb 124 and expiratory limb 126 for ventilated administration of the nitric oxide containing gas flow to the patient 108. The circuit 140 also includes a NO₂ sensor 102 upstream from the conduit 104 and limb 124 junction 125. The sensor 102 sends signals to the NO₂ concentration monitor 118 for monitoring the presence and concentration of any undesirable NO₂.

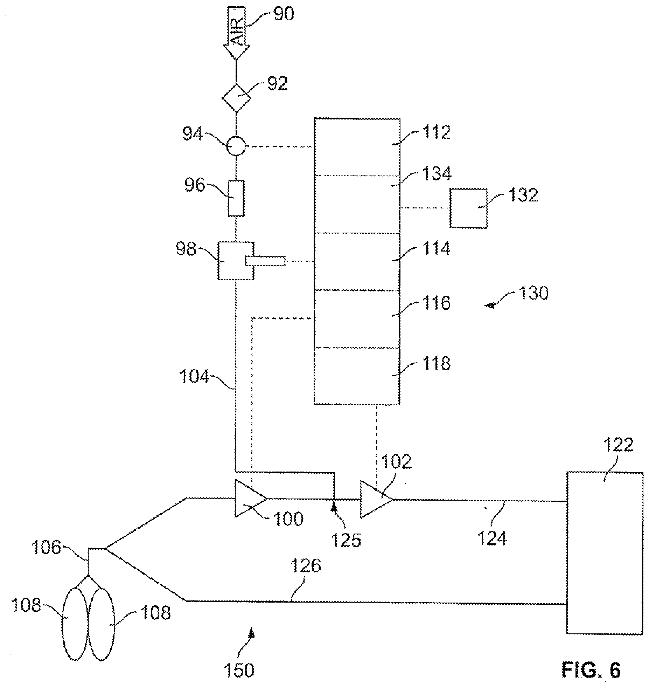

Shown in FIG. 6 is a direct, open-loop, controlled and ventilated nitric oxide generation and delivery system for continuously, spontaneously or intermittently administering a therapeutic dose or concentration of nitric oxide containing gas flow to the patient 108 in need thereof. This system is substantially similar to that shown in FIG. 5. The NO/NO₂ sensor 100 is disposed downstream from the junction 125 between the conduit 104 and inspiratory limb 124. Thus, the NO/NO₂ sensors 100, 102 and corresponding NO/NO₂ sensor monitors 116, 118 monitor and ensure that the predetermined nitric oxide concentration set by the nitric oxide concentration set 132 and corresponding NO concentration set control 134 is accurately administered to the patient 108 in the nitric oxide containing gas flow as well as detecting the presence and concentration of undesirable NO₂. The mechanical ventilator circuit 150 includes the mechanical ventilator 122, inspiratory limb 124, expiratory limb 126, NO/NO₂ sensor 100 and NO/NO₂ sensor 102.

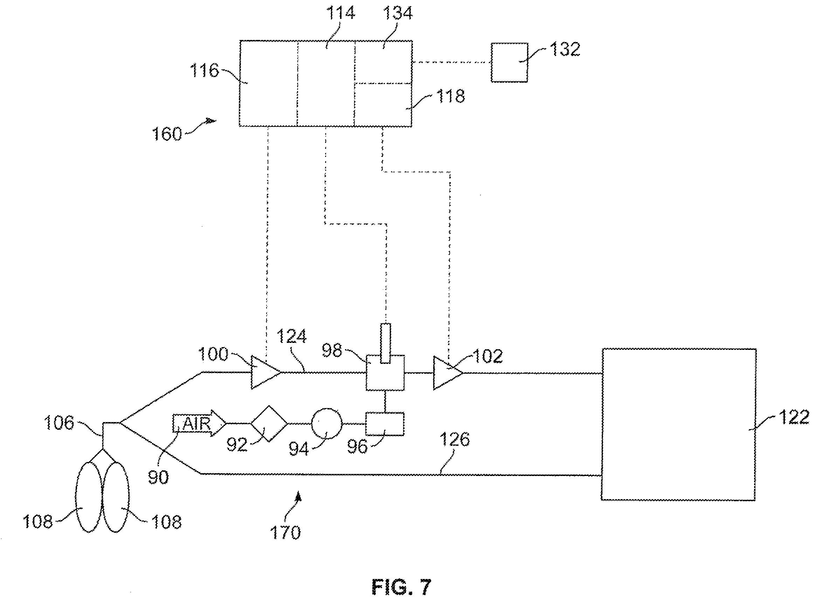

Shown in FIG. 7 is a direct, closed-loop, controlled and ventilated nitric oxide generation and delivery system for continuously, spontaneously or intermittently administering a therapeutic dose or concentration of gaseous nitric oxide to the patient in need thereof 108. In this substantially different configuration, the nitric oxide pulse generator 98 is disposed in the inspiratory limb 124 of the mechanical ventilator circuit 170. Similar to the other configurations, ambient air 90 is filtered of moisture and airborne contaminants by filter 92 and O₂ by the membrane separator 96 with the assistance of pump 94 (pump control not shown) producing filtered and deoxygenated air flow that is fed to the nitric oxide pulse generator 98.

The droplet control 114 controls the frequency of the pulsed nitrite-containing solution contacting the reductant-impregnated/coated hydrophilic nylon tape 36/40 scrolled on the cassette 70 as shown in FIG. 2. The nitric oxide pulses 44 mix with the filtered air flow and pass through the inspiratory limb 124 and the NO/NO₂ sensor 100. The concentration of the nitric oxide at the sensor 100 (i.e., the therapeutic nitric oxide containing gas flow administered to the patient 108) is monitored and maintained by the NO/NO₂ sensors 100, 102 and their corresponding NO/NO₂ sensor monitors 116, 118 in the controller system 160. The presence and concentration of undesirable NO₂ is also detected and reported by NO/NO₂ sensors 100, 102 and their corresponding NO/NO₂ sensor monitors 116, 118 in the controller system 160. The predetermined concentration or dose of nitric oxide in the gas flow is set by the nitric oxide set 132 and its corresponding control 134 in the controller system 160.

There are significant advantages of configuring the mechanical ventilator circuit 170 as having the nitric oxide generator 98 and NO/NO₂ 100, 102 disposed in the inspiratory limb 124. One advantage is faster nitric oxide administration and response to the patient because there is no lag time associated with transporting the gas flow exiting the generator 98 (as depicted in FIGS. 3-6) to the mechanical ventilator circuit 170. Because the nitric oxide is delivered to the patient's lung almost immediately after it is generated (such as in a bedside fashion), there is significantly less risk of generating undesirable side products such as NO₂, thus improving safety and efficacy.

The configuration of the mechanical ventilator circuit 170 shown in FIG. 7 is fully operational at lower peak nitric oxide concentrations as compared to the other mechanical ventilator circuits 120, 140, 150, thus resulting in significantly reducing the risk of generating NO₂. For example, if the patient requires 20 ppm nitric oxide in the gas flow, then the nitric oxide generator need only produce 20 ppm nitric oxide in the gas flow exiting the generator 98 as measured by NO/NO₂ 100. Another advantage is that there is better mixing of the gas flow exiting the generator 98 containing (e.g. 20 ppm nitric oxide) and the filtered air flow.

In contrast, where the nitric oxide generator 98 is not disposed within the mechanical ventilator circuit, the generator must generate a much higher concentration of nitric oxide because the gas flow exiting the generator is generally only around 10% of the air flow in the inspiratory limb of the mechanical ventilator circuit. In which case, the concentration of nitric oxide in the gas flow exiting the reactor would need to be 200 ppm which poses the risk of increased NO₂ formation since its formation is proportional to the square of the NO concentration.

One significant advantage of each medical device and delivery system shown in FIGS. 1-7 is that they are all lightweight, compact and portable, particularly as compared to existing systems having large pressurized nitric oxide tanks and delivery equipment. For example, the instant device and delivery system may be conveniently and quickly moved within a hospital or from one hospital to another hospital, ambulance, helicopter, airplane or other medical transport. The instant device and delivery system also eliminates the need for large, heavy and cumbersome pressurized tanks, which are also present the risk of dangerous accidents attributable to drops, leaks, discharge and/or explosion of its contents to medical staff and patients. The instant device and delivery system also requires significantly less manpower to inventory and transport. In sum, the portability of the instant lightweight and compact medical device and delivery system thereof is a significant and powerful improvement over the current nitric oxide delivery systems utilizing large, heavy pressurized tanks.

In each of the delivery systems shown in FIGS. 3-7, a pulsed injection of gas flow containing a therapeutic dose of nitric oxide may be generated using a manual or automatically controlled trigger known in the art (not shown in FIGS. 3-7). Such pulsed injection is different from the spontaneous breath sensor/trigger/monitor assembly 102, 118 in that the pulsed injection is generated/activated by an operator (e.g., physician, nurse or the like) rather than the patient's breathing.

### EXAMPLES

### EXAMPLE 1

Powdered acids of different acidity (TABLE 1) and reducing capacity, or mixtures at equal weight ratios thereof, were tested for their ability to promote NO release from concentrated aqueous solutions of sodium nitrite. Acids were finely ground and about 5-7 mg placed into wells of a reaction plate contained within a reaction chamber equipped with a microvalve capable of dispensing single 50 nL liquid droplets of sodium nitrite into each well. The reaction chamber was continuously flushed with air at defined flow rates and the NO concentration in the air measured with a SEVERS® NO Analyzer 280i. When NO concentrations exceeded the measurement range of the NO analyzer, the air stream was diluted with a nitrogen stream of defined flow rate prior to measurement and the measured NO concentration corrected for the dilution factor.

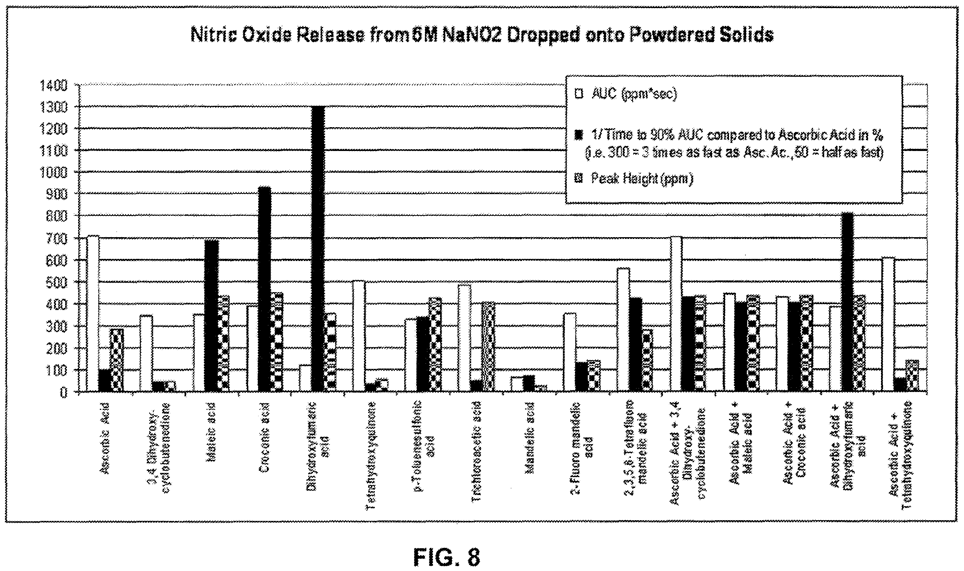

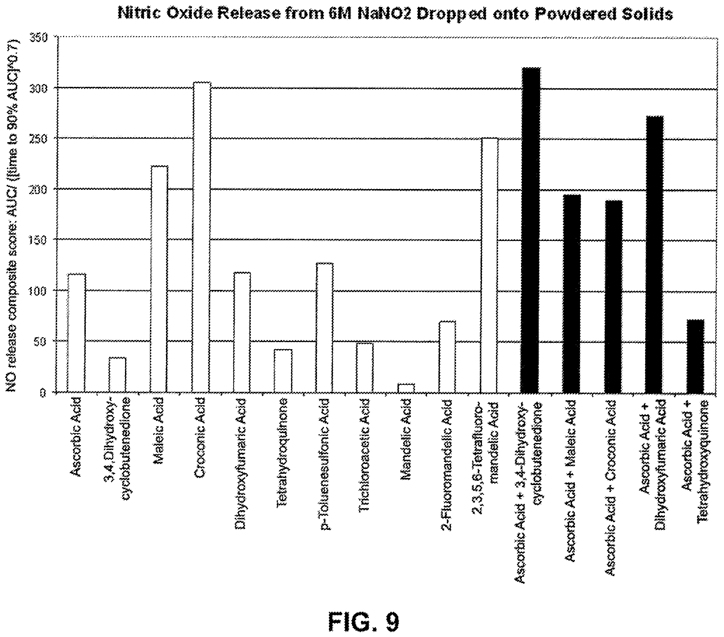

Acids varied greatly in their ability to elicit NO release from droplets of sodium nitrite solution (FIG. 8), as measured by release kinetics, total amount of NO released, and peak NO concentration. Ascorbic acid served as benchmark. An empirical release score composed of peak NO concentration and time required for release of 90% of NO was constructed with the goal to identify acids with fast and large NO release (FIG. 9). Several acids had significantly better release scores than ascorbic acid. Others improved the release score of ascorbic acid when mixed with ascorbic acid.

**TABLE 1**

| **Reductant Name and Structure** | | **pKa** |
|---|---|---|
| Ascorbic Acid | 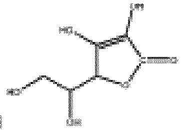 | 4.17 |
| | | 11.6 |
| Squaric Acid | 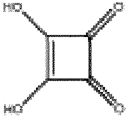 | 0.54 |
| Moleic Acid | 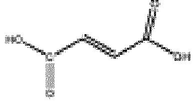 | 1.92 |
| | | 6.23 |
| Croconic Acid | 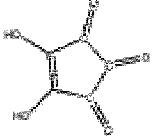 | 0.8 |
| | | 2.24 |
| Dihydroxy-lurnaric Acid | 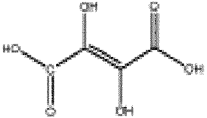 | 1.14 |
| Tetra-hydroxy-quinone | 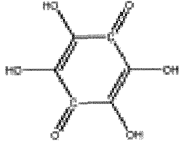 | na |
| p-Toluene-sulfonic acid | 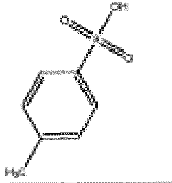 | 0.7 |
| Trichlomecetic acid | 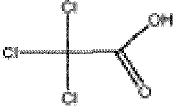 | 0.77 |
| Mandelic acid | 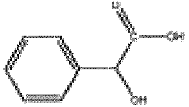 | 3.37 |
| 2-Fluoro-mandelic acid | 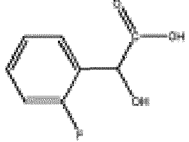 | <3.4 |
| 2,3,5,6-Tetrafluoromandelic acid | 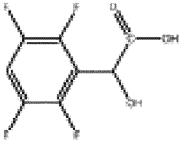 | <<3.4 |

### EXAMPLE 2

Procedure for synthesis of NO by reaction of croconic acid with sodium nitrite and comparison of NO production from this reaction to that from the reaction of sodium nitrite with ascorbic acid.

50 ml of 6M NaNO₂ stock solution was prepared and degassed for 15 minutes. 10 mL of the stock solution was filled into a liquid reservoir feeding a microvalve capable of dispensing single 50 nL liquid droplets sequentially into each of six wells of a reaction plate contained within a reaction chamber. The air flow rate through the reaction chamber was set to 500 mL/minute. The air flow was diluted with nitrogen gas to a flow rate of 1586 mL/minute to allow measurement of the entire NO peak with a SIEVERS® NO Analyzer 280i. The NO analyzer was calibrated using a 45 ppm NO calibration gas prior to the experiment.

Three wells of the 6-well reaction plate were each filled with 7 mg of finely ground croconic acid powder and three wells were each filled with 7 mg of finely ground ascorbic acid powder. The microvalve fired a 50 nL droplet into each well. The timecourse of NO production was measured with the NO Analyzer. The following time parameters were analyzed for each NO peak: AUC, time to reaching 90% of the AUC, and maximum peak NO concentration. The results are shown in FIG. 1 1 and Table 2.

**TABLE 2**

| | Ascorbic Acid Powder | Croconic Acid Powder |
|---|---|---|
| Time to 90% AUC (sec) | 13.98 | 1.1 |
| Maximum peak concentration (ppm) | 156.4 | 711.5 |
| AUC (ppm·sec) | 304.16 | 313.2 |

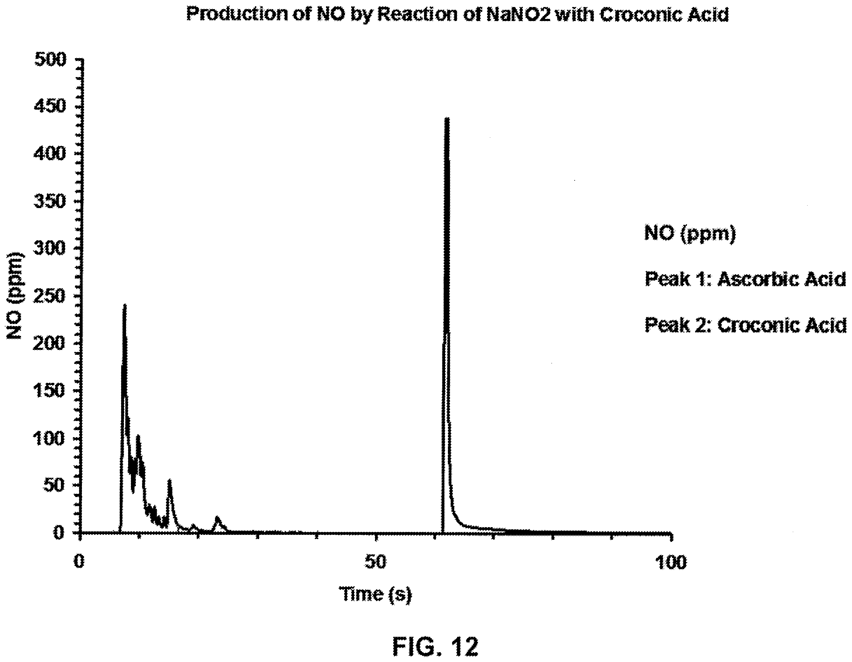

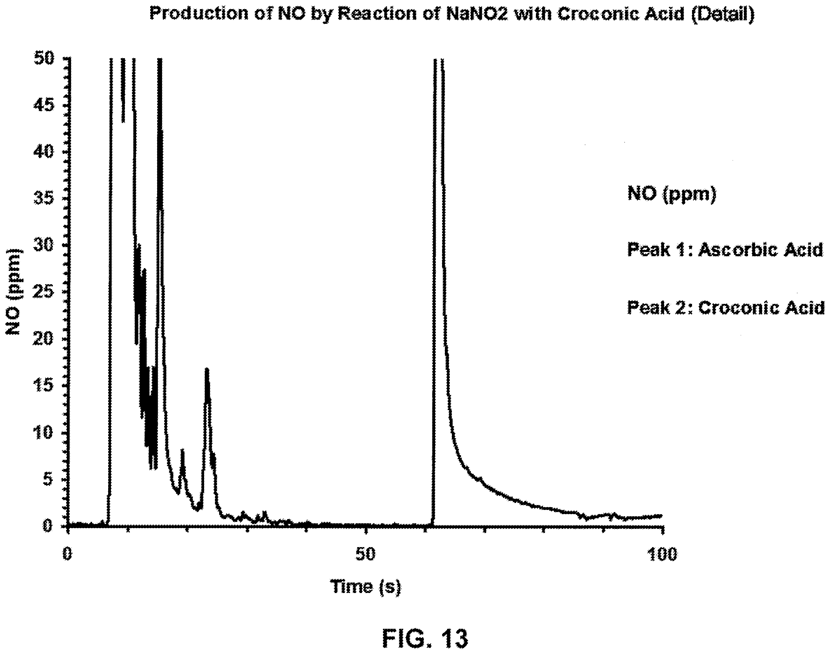

Croconic Acid exhibited superior NO release kinetics over ascorbic acid while releasing similar quantities of NO, as demonstrated by the quick response time, the high peak NO concentration and the smooth decay reflecting the gradual and controlled cessation of the chemical reaction. In contrast, NO release by ascorbic acid is slow, protracted and irregular (FIG. 12 and FIG. 13).

### EXAMPLE 3

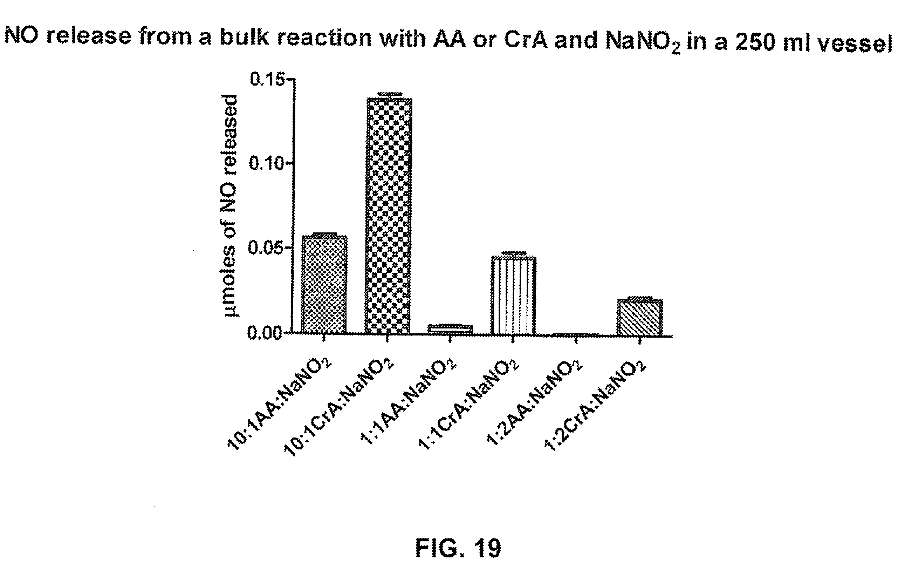

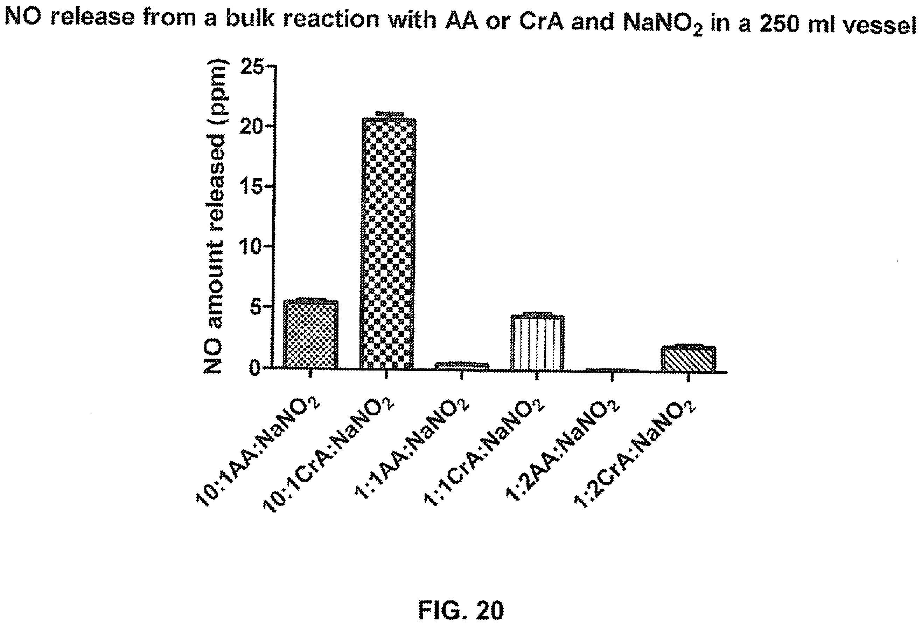

Procedure for the NO synthesis from a bulk reaction of croconic acid or ascorbic acid and sodium nitrite. Anoxic 1 mM stock solutions of NaNO₂, croconic acid, and ascorbic acid were prepared in a nitrogen-filled glove box. A 250 mL round bottom flask containing a stir bar was purged with nitrogen gas for 10 minutes, 10 mL of croconic acid or ascorbic acid solution added followed by 1 mL of NaNO₂ solution. The resulting solution was stirred for 2 minutes at room temperature, the resulting NO gas flushed with nitrogen and the NO concentration determined using the bag analysis program on the SEVERS® NO Analyzer 280i. The results are shown in FIGS 19 and 20.

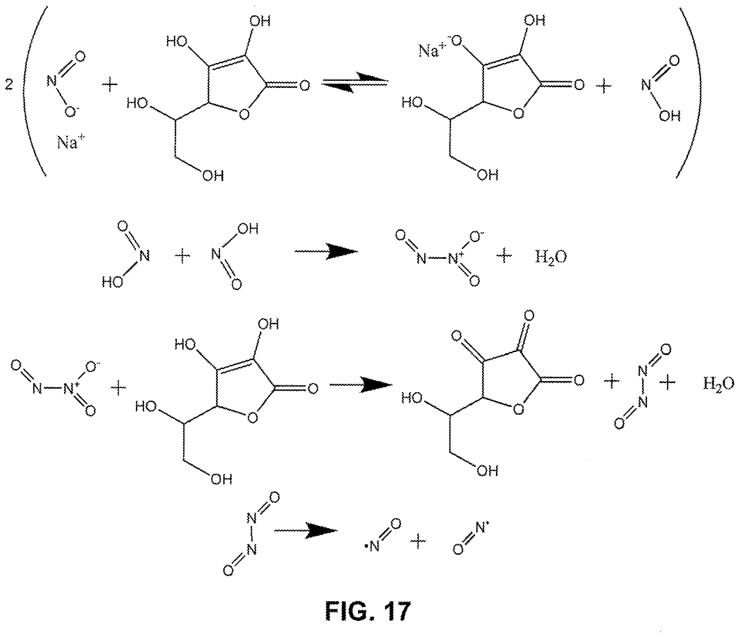

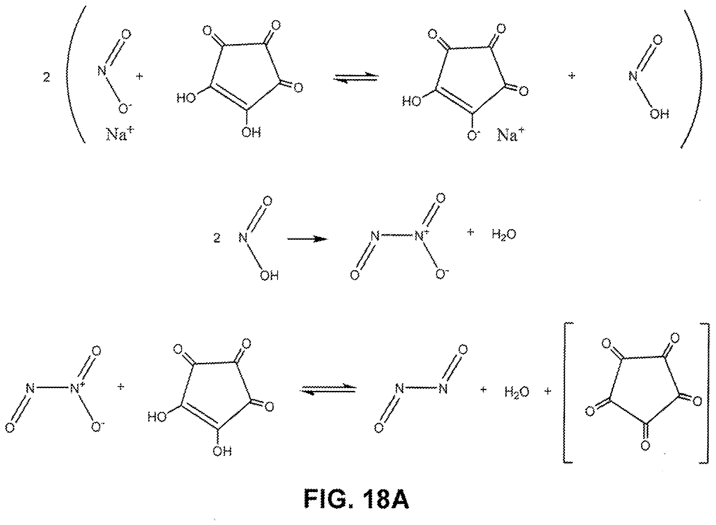

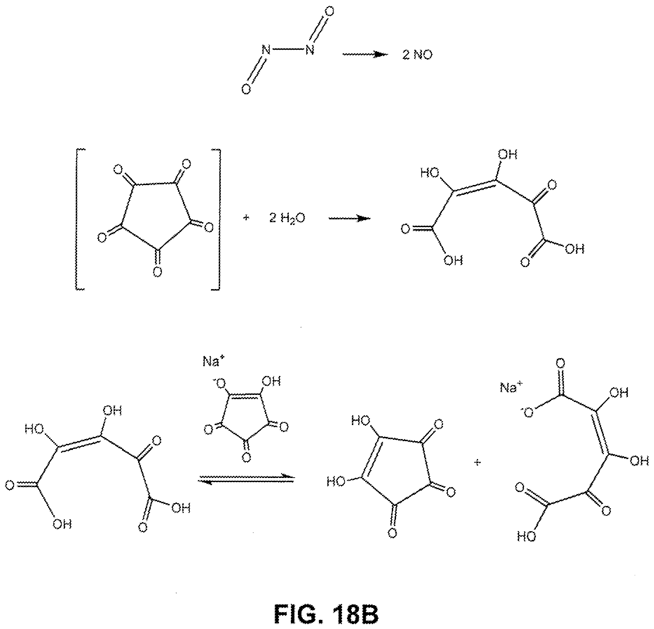

Croconic acid releases more NO than ascorbic acid on a molar basis, presumably because the underlying reaction stoichiometry is different (FIGS. 17 and 18). Similar to the reaction of ascorbic acid, one equivalent of croconic acid may react as reductant. However, in contrast to ascorbic acid, only one equivalent of croconic acid is consumed as acid as the oxidized croconic acid may regenerate one equivalent of croconic acid during the reaction.

### EXAMPLE 4

The following procedures describe the coating of MAGNA® hydrophobic nylon tape with suspensions of croconic acid for use with the NO generation device shown in FIG 2.

The following materials were used in the experiment. Croconic acid, TCI America, CAS#: 488-86-8, Cat. #: C1483-5G; Methanol, Fisher Chemical, CAS#: 67- 56-1, Cat. #: A456-4; Cornstarch, Spectrum, CAS#: 9005-25-8, Cat. #: S1552-500GM; and, Ethanol, Acros Organics, CAS#: 64-17-5, Cat. #: AC61509-001 0.

### EXAMPLE 4A

10 grams of croconic acid were milled for 5 minutes using a bench top micromill and sifted through a 32 µm sieve. 100 mg of milled acid were suspended into 1 mL of absolute MeOH supplemented with 2% v/v cornstarch and 0.05% v/v glycerol. The yellow-orange suspension was spread evenly as a single coat using an acid resistant brush onto a 5 mm by 18 inch MAGNA® hydrophobic nylon tape of 1 .2 µm pore size. The tape was air-dried for 30 min by suspending it horizontally at both ends. The croconic acid formed a dark yellow slightly powdery coating on top of the nylon membrane; the average surface density of croconic acid deposited on the tape was 2.66 mg/cm².

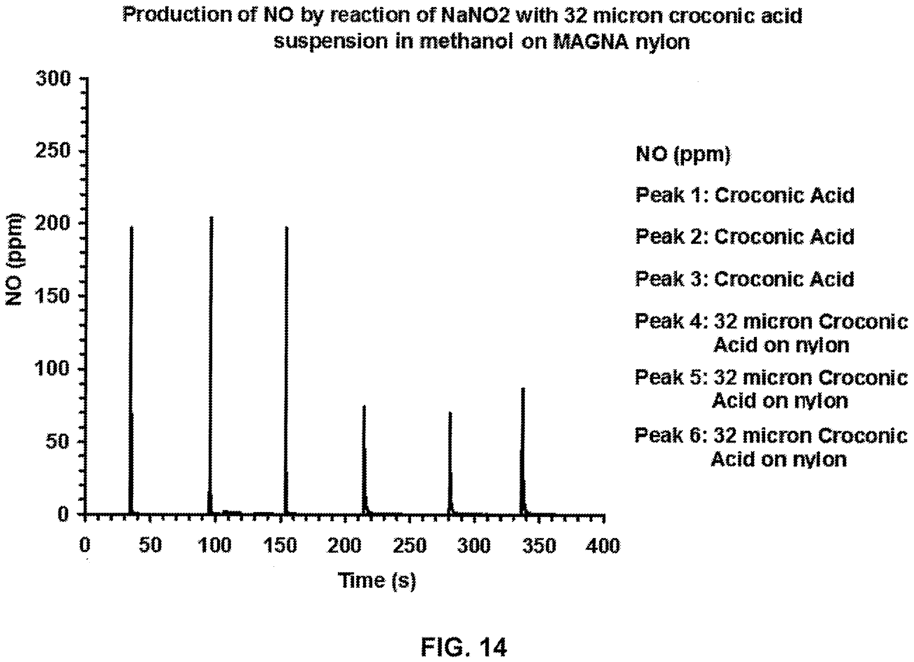

NO release in response to single 50 nL droplets of 6M NaNO₂ is shown in FIG 14 and TABLE 3, compared to the NO release from croconic acid powder. The croconic acid coated tape produced 85.3% of the NO compared to the bulk croconic acid powder, based upon the AUC.

**TABLE 3**

| | Particulate Croconic Acid | Croconic acid-impregnated Magna® nylon membrane |
|---|---|---|
| Time to 90% AUC (sec) | 0.79 | 3.7 |
| Maximum peak concentration (ppm) | 657.26 | 255.98 |
| AUC (ppm·sec) | 258.23 | 221.23 |

### EXAMPLE 4B

Particulate croconic acid of particle size < 20µm produced by dry-milling for 45 min was formulated as 500 mg/ml suspension in USP ethanol by 10 min of sonication with additives of 0.05% v/v glycerol and 2% v/v starch and coated onto MAGNA® hydrophobic nylon tape in two sequential air-dried coats, yielding an average croconic acid surface density of 4.42 mg/cm².

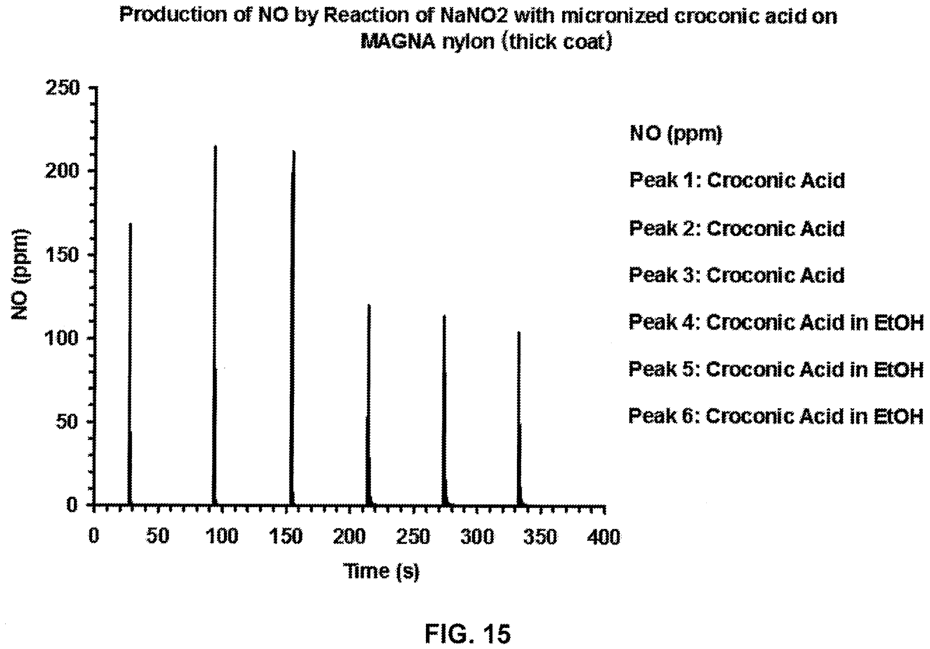

NO release in response to single 50 nL droplets of 6M NaNO₂ is shown in FIG. 15 and TABLE 4, compared to the NO release from particulate croconic acid. The croconic acid coated tape yielded 93.9% of the NO compared to the bulk croconic acid powder.

**TABLE 4**

| | Particulate Croconic Acid | Croconic acid-impregnated Magna® nylon membrane |
|---|---|---|
| Time to 90% AUC (sec) | 0.85 | 2.18 |
| Maximum peak concentration (ppm) | 655.05 | 370.22 |
| AUC (ppm·sec) | 293.30 | 275.65 |

### EXAMPLE 4C

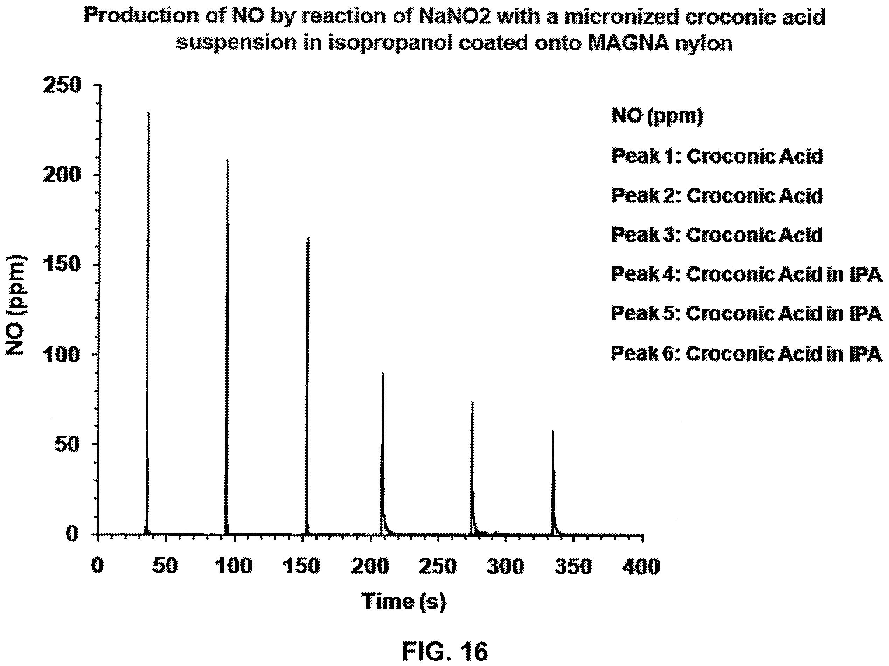

In another example, the procedure of the preceding paragraph was repeated by suspending 350 mg of milled croconic acid in 1 mL of isopropanol and preparing two sequential air-dried coats on the MAGNA® hydrophobic nylon strip yielding a croconic acid deposited as a dark yellow coating on the nylon membrane. NO release in response to single 50 nL droplets of 6M NaNO₂ is shown in FIG. 16 and TABLE 5, compared to the NO release from particulate croconic acid. The croconic acid coated tape yielded 84.7% of the NO compared to the bulk croconic acid powder.

**TABLE 5**

| | Particulate Croconic Acid | Croconic acid-impregnated Magna® nylon membrane |
|---|---|---|
| Time to 90% AUC (sec) | 0.77 | 5.37 |
| Maximum peak concentration (ppm) | 688.42 | 243.86 |
| AUC (ppm·sec) | 280.37 | 237.37 |

### EXAMPLE 5

Croconic acid-coated MAGNA hydrophic nylon tape was mounted in the device of FIG. 2 and 6M sodium nitrite-containing fluid sprayed onto the tape from a piezoelectric inkjet dispensing liquid droplets of volumes of about 10 pL per droplet. A stepper motor advanced the tape in proportion to the rate of nitrite deposition at three different rates to expose fresh tape to the liquid droplets. Droplet production was controlled by a computer at rates between 30 and 400 nL/inch of tape. NO was carried by airflow through the reaction chamber to a SIEVERS® NO Analyzer 280i and an NO₂ gas detector.

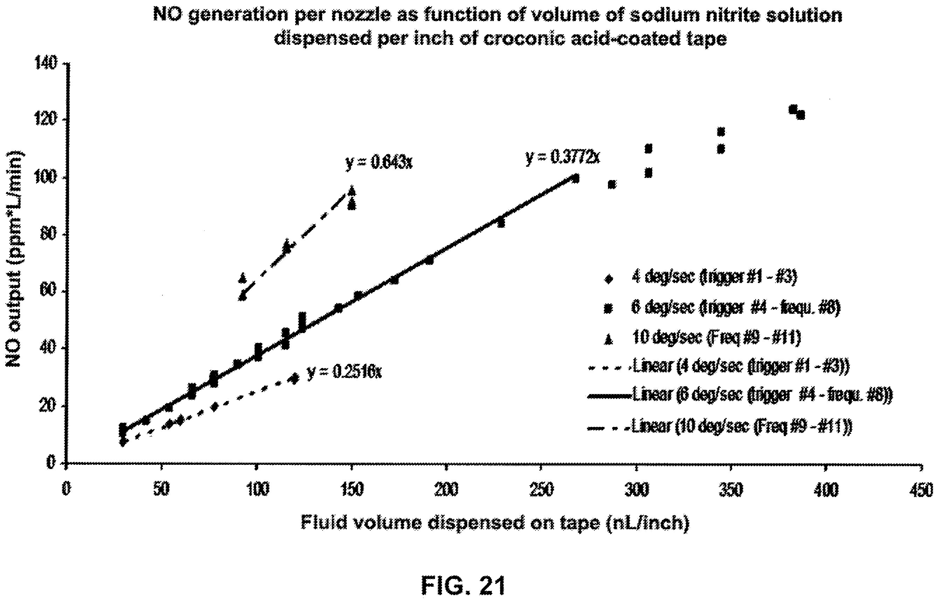

NO release as a function of varying tape speed and sodium nitrite deposition onto the tape is shown in FIGS 20 and 21. NO concentrations in the airstream varied between 5 and 120 ppm*L/min; NO release was strictly proportional over wide range of sodium nitrite amounts dispensed; further control of NO release was afforded by varying tape speed. Less than 2% NO₂ was produced by the device.

## Claims

1. An apparatus for making pharmaceutical nitric oxide gas comprising:
an enclosure defining a reactor chamber having a gas inlet port and a gas outlet port, the inlet port adapted to receive an air flow, and, the gas outlet port adapted to discharge a gas flow;
a tape constructed from:
a porous hydrophilic polymer substrate having pores, an average pore size, and, a substrate thickness, and,
a particulate reductant disposed within the pores and on a surface of the tape forming a film, the particulate reductant having an average particle size, and, the film having a surface density of particulate reductant and a film thickness,
the tape disposed within the reaction chamber, and, the tape wound around first and second cylinders of a spooling mechanism,
the spooling mechanism adapted to spool the tape within the reaction chamber; and,
a liquid pulsing device adapted to pulse liquid droplets of a liquid source comprising water, NO₂⁻, viscosity agent, and, non-detergent surfactant onto the tape generating pulses of the nitric oxide gas,
wherein the reductant comprises one or more compounds selected from the following structures: 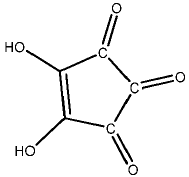 (croconic acid), 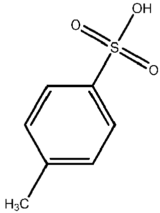 (p-toluenesulfonic acid), and 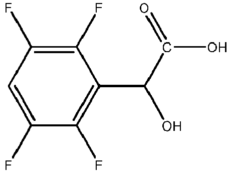 (2,3,5,6-tetrafluoromandelic acid).

2. The apparatus of claim 1, wherein the liquid pulsing device is in liquid communication with a liquid inlet port disposed on the enclosure, and wherein the air flow further comprises N₂ and/or O₂.

3. The apparatus of claim 1, wherein the spooling mechanism is a cassette.

4. The apparatus of claim 1, wherein the liquid pulsing device is a piezoelectric ink jet printhead.

5. The apparatus of claim 4, wherein the piezoelectric ink jet printhead is adapted to generate liquid droplets having a volume in the range of 10-100 picoliters.

6. The apparatus of claim 4, wherein the piezoelectric ink jet printhead is adapted to generate pulses of droplets at a frequency up to 12 MHz.

7. The apparatus of claim 5, wherein the piezoelectric ink jet printhead is adapted to generate 10-100 nL per pulse of the liquid source.

8. The apparatus of claim 5, wherein the piezoelectric ink jet printhead is adapted to generate 25-75 nL per pulse of the liquid source.

9. The apparatus of claim 5, wherein the piezoelectric ink jet printhead is adapted to generate around 48 nL per pulse of the liquid source.

10. The apparatus of claim 1,
wherein the porous hydrophilic polymer comprises a polyamide nylon;
wherein the water is deionized and millipore-filtered;
wherein the non-detergent surfactant comprises a nonylphenol ethoxylate; and, wherein the viscosity agent comprises polyethylene glycol having a molecular weight in the range of 3500-20,000.

11. The apparatus of claim 10,
wherein the reductant comprises croconic acid according to the structure 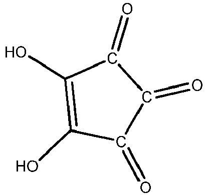 or a salt thereof; wherein the non-detergent surfactant comprises a nonylphenol ethoxylate 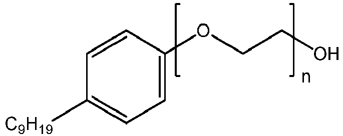 according to the structure having an average molecular weight around 616; and, wherein the viscosity agent comprises polyethylene glycol having a molecular weight in the range of around 4400 to 4600.

## Patentansprüche

1. Vorrichtung zur Herstellung von pharmazeutischem Stickoxidgas, umfassend:
ein Gehäuse, das eine Reaktorkammer mit einer Gaseinlassöffnung und einer Gasauslassöffnung definiert, wobei die Einlassöffnung zur Aufnahme eines Luftstroms und die Gasauslassöffnung zur Abgabe eines Gasstroms ausgelegt sind;
ein Band, konstruiert aus:
einem porösen hydrophilen Polymersubstrat mit Poren, einer durchschnittlichen Porengröße und einer Substratdicke, und,
einem teilchenförmigen Reduktionsmittel, das innerhalb der Poren und auf einer Oberfläche des Bandes angeordnet ist, sodass es einen Film bildet,
wobei das teilchenförmige Reduktionsmittel eine durchschnittliche Teilchengröße aufweist, und wobei der Film eine Oberflächendichte des teilchenförmigen Reduktionsmittels und eine Filmdicke aufweist,
wobei das Band innerhalb der Reaktionskammer angeordnet ist und um den ersten und zweiten Zylinder eines Spulmechanismus gewickelt ist,
wobei der Spulmechanismus angepasst ist, um das Band innerhalb der Reaktionskammer zu spulen; und,
wobei eine Flüssigkeitspulsvorrichtung angepasst ist, um Flüssigkeitströpfchen einer Flüssigkeitsquelle, die Wasser, NO₂⁻,
Viskositätsmittel und nicht-detergierendes Tensid umfasst, gepulst auf das Band aufzubringen, um so Pulse des Stickoxidgases zu erzeugen,
wobei das Reduktionsmittel eine oder mehrere Verbindungen umfasst, die aus den folgenden Strukturen ausgewählt sind: 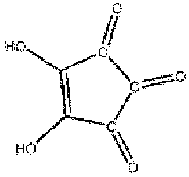 (Croconsäure) 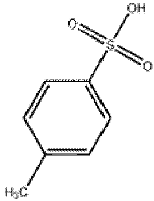 (p-Toluolsulfonsäure) und 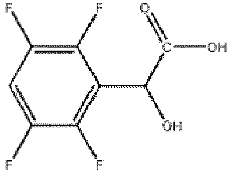 (2,3,5,6-Tetrafluormandelsäure).

2. Vorrichtung nach Anspruch 1, wobei die Flüssigkeitspulsvorrichtung in Flüssigkeitsverbindung mit einer am Gehäuse angeordneten Flüssigkeitseinlassöffnung steht, und wobei der Luftstrom ferner N₂ und/oder O₂ umfasst.

3. Vorrichtung nach Anspruch 1, wobei der Spulmechanismus eine Kassette ist.

4. Vorrichtung nach Anspruch 1, wobei die Flüssigkeitspulsvorrichtung ein piezoelektrischer Tintenstrahldruckkopf ist.

5. Vorrichtung nach Anspruch 4, wobei der piezoelektrische Tintenstrahldruckkopf angepasst ist, um Flüssigkeitströpfchen mit einem Volumen im Bereich von 10-100 Picolitern zu erzeugen.

6. Vorrichtung nach Anspruch 4, wobei der piezoelektrische Tintenstrahldruckkopf angepasst ist, um Pulse von Tropfen mit einer Frequenz von bis zu 12 MHz zu erzeugen.

7. Vorrichtung nach Anspruch 5, wobei der piezoelektrische Tintenstrahldruckkopf angepasst ist, um 10-100 nl pro Puls der Flüssigkeitsquelle zu erzeugen.

8. Vorrichtung nach Anspruch 5, wobei der piezoelektrische Tintenstrahldruckkopf angepasst ist, um 25-75 nl pro Puls der Flüssigkeitsquelle zu erzeugen.

9. Vorrichtung nach Anspruch 5, wobei der piezoelektrische Tintenstrahldruckkopf angepasst ist, um etwa 48 nl pro Puls der Flüssigkeitsquelle zu erzeugen.

10. Vorrichtung nach Anspruch 1,
wobei das poröse hydrophile Polymer ein Polyamid-Nylon umfasst; wobei das Wasser deionisiert und Millipore-filtriert ist;
wobei das nicht-detergierende Tensid ein Nonylphenolethoxylat umfasst; und,
wobei das Viskositätsmittel Polyethylenglykol mit einem Molekulargewicht im Bereich von 3500-20.000 umfasst.

11. Vorrichtung nach Anspruch 10, wobei das Reduktionsmittel Croconsäure
umfasst gemäß der Struktur 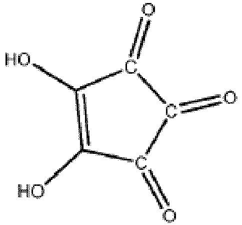 oder ein Salz davon;
wobei das nicht-detergierende Tensid ein Nonylphenolethoxylat umfasst 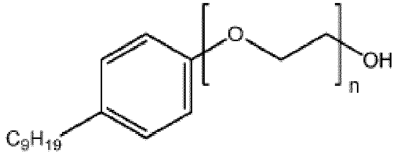 gemäß dieser Struktur mit einem durchschnittlichen Molekulargewicht um 616; und, wobei das Viskositätsmittel Polyethylenglykol mit einem Molekulargewicht im Bereich von etwa 4400 bis 4600 umfasst.

## Revendications

1. Appareil pour produire du gaz d'oxyde nitrique pharmaceutique comprenant:
une enceinte définissant une chambre formant réacteur ayant un orifice d'entrée de gaz et un orifice de sortie de gaz, l'orifice d'entrée adapté pour recevoir un courant d'air et l'orifice de sortie de gaz adapté pour évacuer un courant de gaz;
un ruban construit à partir de:
un substrat à base de polymère hydrophile poreux ayant des pores, une dimension de pore moyenne et une épaisseur de substrat, et
un réducteur particulaire disposé dans les pores et sur une surface du ruban formant un film, le réducteur particulaire ayant une dimension de particule moyenne, et le film ayant une densité de surface de réducteur particulaire et une épaisseur de film,
le ruban disposé dans la chambre formant réacteur et le ruban enroulé autour de premier et second cylindres d'un mécanisme de bobinage,
le mécanisme de bobinage adapté pour bobiner le ruban dans la chambre formant réacteur;
et
un dispositif de pulsion de liquide adapté pour pulser des gouttelettes de liquide d'une source de liquide comprenant de l'eau, NO₂⁻, un agent de viscosité et un tensioactif non détergent sur le ruban produisant des impulsions du gaz d'oxyde nitrique,
où le réducteur comprend un ou plusieurs composés choisis parmi les structures suivantes: 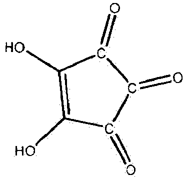 (acide croconique), 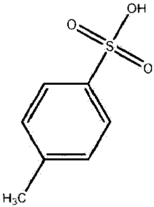 (acide p-toluènesulfonique), et 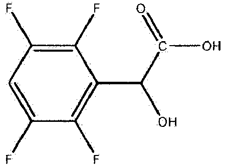 (acide 2,3,5,6-tétrafluoromandélique).

2. Appareil selon la revendication 1, où le dispositif de pulsion de liquide est en communication liquide avec un orifice d'entrée de liquide disposé sur l'enceinte, et où le courant d'air comprend en outre N₂ et/ou O₂.

3. Appareil selon la revendication 1, où le mécanisme de bobinage est une cassette.

4. Appareil selon la revendication 1, où le dispositif de pulsion de liquide est une tête d'impression à jet d'encre piézoélectrique.

5. Appareil selon la revendication 4, où la tête d'impression à jet d'encre piézoélectrique est adaptée pour produire des gouttelettes de liquide ayant un volume dans la plage de 10-100 picolitres.

6. Appareil selon la revendication 4, où la tête d'impression à jet d'encre piézoélectrique est adaptée pour produire des impulsions de gouttelettes à une fréquence de jusqu'à 12 MHz.

7. Appareil selon la revendication 5, où la tête d'impression à jet d'encre piézoélectrique est adaptée pour produire 10-100 nL par impulsion de la source de liquide.

8. Appareil selon la revendication 5, où la tête d'impression à jet d'encre piézoélectrique est adaptée pour produire 25-75 nL par impulsion de la source de liquide.

9. Appareil selon la revendication 5, où la tête d'impression à jet d'encre piézoélectrique est adaptée pour produire environ 48 nL par impulsion de la source de liquide.

10. Appareil selon la revendication 1,
où le polymère hydrophile poreux comprend un nylon de type polyamide;
où l'eau est désionisée et filtrée sur millipore;
où le tensioactif non détergent comprend un produit d'éthoxylation de nonylphénol; et
où l'agent de viscosité comprend un polyéthylèneglycol ayant une masse moléculaire dans la plage de 3500-20000.

11. Appareil selon la revendication 10,
où le réducteur comprend de l'acide croconique selon la structure 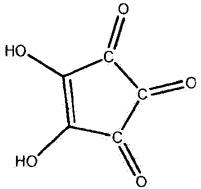 ou un sel de celui-ci;
où le tensioactif non détergent comprend un produit d'éthoxylation du nonylphénol 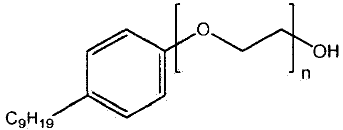 selon la structure ayant une masse moléculaire moyenne d'environ 616; et
où l'agent de viscosité comprend un polyéthylèneglycol ayant une masse moléculaire dans la plage d'environ 4400 à 4600.
